# EUROPEAN PATENT APPLICATION

(11) **EP 1 920 788 A1**
(43) Date of publication of application: **14.05.2008**
(21) Application number: 06425772.8
(22) Date of filing: 13.11.2006
(51) Int. Cl.: A61L 27/24, A61L 27/36, A61L 27/38

(54) **Odontologic and osteogenic implants and scaffolds containing mesenchymal stem cells**

(71) Applicant: Tredici, Giovanni, 20052 Monza MI (IT); Carini, Fabrizio, 20052 Monza MI (IT); Bandera, Adriano, 20121 Milano (IT); Baldoni, Marco, 20052 Monza MI (IT)
(72) Inventor: Tredici, Giovanni, 20052 Monza MI (IT); Carini, Fabrizio, 20052 Monza MI (IT); Bandera, Adriano, 20121 Milano (IT); Baldoni, Marco, 20052 Monza MI (IT)
(74) Representative: Tansini, Elio Fabrizio

(57) **Abstract**

The present invention relates to a scaffold containing mesenchymal stem cells and a plasma rich in platelets (PRP). Furthermore, the present invention relates to a scaffold including a plurality of adjacent portions in a mutual contact to define a single structure, said scaffold contains mesenchymal stem cells and, optionally, a plasma rich in platelets (PRP).

## Description

The present invention relates to a scaffold containing mesenchymal stem cells and a plasma rich in platelets (PRP). Furthermore, the present invention relates to a scaffold comprising of a plurality of adjacent portions in a mutual contact to define a single structure, said scaffold containing mesenchymal stem cells and, optionally, a plasma rich in platelets (PRP).

### KNOWN ART

The parodontal disease is a chronic inflammatory phenomenon with a microbiologic etiology and has a high incidence in the population. This disease has serious consequences for the parodontal apparatus to which a possible reabsorption of the alveolar bone, the periodental ligaments, the cement and the gingivae can follow. Finally, the parodontal disease can bring to the loss of the tooth itself. As such, the parodontal disease represents a group of bone degeneration pathologies which can be treated with implants for replacing the defects of the bone tissue.

The treatment of said pathology presents some difficulties of a greater importance with respect to other therapies applied to cases of bone degeneration pathologies, as the treatment has also to include the creation and the adaptation of the different tissues surrounding the new bone material which, in case of the parodontal disease, are complex tissues and include: the alveolar bone, a new cement layer and a new periodontal ligament.

The known treatments used in the art are based on the regeneration of the bone tissue, the regeneration of the enamel matrix or are based on the use of growth factors, but objectively do not produce satisfactory results.

Some studies have already been developed in order to find a possible use of a cell therapy for regenerating the tissue. The use of mesenchymal stem cells for the regeneration of bone and dental tissue has already been looked at. The mesenchymal stem cells have been studied thanks to their multi-strong abilities of differentiation in mesengenic (osteogenic, chondrogenic and adipogenic) cell lines, their ease in being isolated from aspirate of bone marrow, their high expansion ability *ex vivo* (Bruder et al. 1997, Colter et al. 2000) and their immunological properties (Le Blank et al. 2003).

Some pre-clinical models have used a three-dimensional structure as a framework, herein called scaffold, in/on which the stem cells have been introduced to be consolidated and induced to regenerate the bone tissue lacking in other sites of exclusive orthopedic interest (Cancedda et al. 2003 and Caplan et al. 2005) or lacking bone tissue for the tooth implant (De Kok et al 2003 and Kawaguchi et al 2004). The material of said scaffold must meet some requirements of biomimeticity and biodegradability to be effective. Some models in the art have already employed the same material used in nature: collagen.

It is also known how to induce the mesenchymal cells in an osteogenic sense (ref. Yoshikawa et al 1996 and

Ohgushi et al 1996): for example, by exposing the cells to an "osteogenic" medium, that is a medium containing a factor promoting the differentiation in an osteogenic sense, such as for example dexametasone.

A crucial problem consists of the vascularization of both the scaffold, containing the mesenchymal cells, and the bone tissue in the course of formation.

There remains therefore the need of being able to provide a scaffold containing the mesenchymal cells which does not have the drawbacks of the known art.

In particular, there remains therefore the need of being able to provide a scaffold containing the mesenchymal cells suitable for being implanted in the bone tissue and in the parodontal one, besides being easy to prepare/construct and to use.

Still more particularly, there remains the need of being able to provide a scaffold containing the mesenchymal cells capable of promoting the vascularization of the same and the surrounding tissue.

### Description

With the present invention, a scaffold containing mesenchymal stem cells (MSC), capable of promoting the vascularization processes of the scaffold itself is proposed. The scaffold allows the reparation of the bone defect with a similar/equal bone tissue. The Applicant has found that the vascularization in a scaffold can be encouraged/promoted by using a plasma rich in platelets (PRP).

In an embodiment of the present invention a PRP rich in growth factors, which promotes the growth of blood vessels, is employed. The PRP comprises:
- vascular endothelial growth factor (VEGF) and/or
- platelet-derived growth factor (PDGF) and/or
- transforming growth factor (TGF).

In a preferred embodiment, the PRP is additioned to the scaffold after the MSC have been introduced in the same.

The PRP is arranged on the outer surface of the scaffold. The PRP stimulates the formation of the vessels and the re-vascularization of the bone tissue in the course of re-formation by acting both on the blood vessels, existing in the bone tissue surrounding the bone defect, and promoting the penetration of the vessels in the tissue in course of formation in the scaffold.

The PRP is preferably obtained from the patient through collection of a small quantity of blood; which is then subjected to a centrifugation so as to isolate the platelet fraction and the plasma.

In an embodiment of the present invention, the PRP is activated with an anti-hemorrhagic and/or calcium gluconate. The anti-hemorrhagic can contain the active substance batroxobine, such as for example Botropase^{®}. In a preferred embodiment, the anti-hemorrhagic and/or calcium gluconate are added to the PRP with a volumetric/quantitative ratio of 1:2.5 anti-hemorrhagic and/or calcium gluconate:PRP, and a gelatinous mass is obtained after a few minute (5'-10'). The gelatinous mass can be arranged into contact with the scaffold surface.

The scaffold is a framework which has to meet the requirements of biomimeticity and biodegradability such to allow a gradual but complete replacement of it with the regenerated tissue, without causing adverse reactions.

Preferably, the scaffold is made of a collagen material, type I.

The scaffold is preferably structured so as to be porous and still more preferably it contains pores with a diameter between 100 and 500 microns, preferably between 200 and 400; still more preferably, between 250 and 350 microns.

A preferred embodiment of said collagen scaffold foresees the use of a porous scaffold of the Gingistat^{®} type.

The scaffold can have volumes that vary depending on the purpose for which it is used. The scaffold can have a total volume and dimensions lower or equal to the volume and dimensions of the bone defect. For example, in order to produce a dental implant, it can be sufficient to use a scaffold with a volume between 100 and 300 mm³, but in presence of more marked bone defects the dimensions can also be greater, for example 900-1000 mm³.

The scaffold can assume different types of three-dimensional geometric forms, selected for example from: cube, cylinder, cylinder with a hexagonal base, parallelepiped, prism with a triangular base, pyramid with a squared or rectangular base.

The number of MSC introduced in the scaffold depends on the scaffold volume and the ratio between the two can vary. It is preferable to have a quantitative ratio between 5000-10000 MSC/mm³ of scaffold, still more preferably between 7000-9000 MSC/mm³; for example, between 7500-8500 MSC/mm³.

In an embodiment, in a dental implant comprising the scaffold with an approximately cubic form 5x5x5 mm, 1x10⁶ MSC/125 mm³ of scaffold are preferably used.

The mesenchymal stem cells (MSC) are preferably collected from the iliac crest of the bone marrow and isolated through separation on a Ficoll gradient. The effectiveness of the isolation process is determined by the Fluorescence-activated cell-sorting (FACS) method. The selected cells must express the antigens HLA-ABC, CD90 and CD105 on the cell surface and must not express the antigens CD33, CD34 and HLA-DR on the cell surface.

The MSC are preferably placed in a medium for promoting their expansion after the isolation. The MSC are subsequently introduced in an osteogenic medium in order to promote an osteogenic differentiation and subsequently introduced in a scaffold and cultivated in vitro, thus allowing the initial differentiation in a bone sense.

The scaffold containing said MSC and having the PRP on its surface advantageously promotes/encourages the vascularization as an implant for a bone or dental defect.

In another embodiment, the scaffold subject of the present invention includes a plurality of portions of scaffold adjacent with each other and in mutual contact in order to define a single structure.

Each portion includes said mesenchymal stem cells (MSC) or, alternatively, said mesenchymal stem cells (MSC) and said PRP.

The dimensions of the single scaffold portions are such so as to allow the distribution and the penetration of the MSC within the volume of each portion. The plurality of scaffold portions add up to give the total dimension and the form of the bone defect.

The arrangement of a plurality of portions in a mutual contact allows to promote the formation of new bone throughout the whole scaffold.

The sum of the portions forms a scaffold which can have varying volumes depending on the aim for which it is used. Generally, it is dimensioned on the total volume of the bone defect. For example, in order to produce a dental implant, it can be sufficient to use a scaffold with a volume between 100 and 300 mm³, but in the presence of more marked bone defects the dimensions can also be greater, for example 900-1000 mm³.

The single portions can have different types of three-dimensional geometric forms, selected for example from: cube, cylinder, cylinder with a hexagonal base, parallelepiped, prism with a triangular base, pyramid with a squared or rectangular base.

The sum, according to preference, of the single portions can give a scaffold having a three-dimensional geometric forms, selected for example from: cube, cylinder, cylinder with a hexagonal base, parallelepiped, prism with a triangular base, pyramid with a squared or rectangular base.

The number of MSC introduced in the scaffold depends on the scaffold volume and the ratio between the two can vary.

It is preferable to have a quantitative ratio between 5000-10000 MSC/mm³ of scaffold, still more preferably between 7000-9000 MSC/mm³; for example, between 7500-8500 MSC/mm³.

The scaffold portions can be, for example, of a cubic or parallelepiped form.

For example, a scaffold with a cubic form can have dimensions between 1x1x1 mm and 10x10x10 mm.

For example, in case of a scaffold with dimensions of 6x6x6 mm, eight cubic portions with dimensions of 3x3x3 can be placed into mutual contact.

For example, in case of a 9x9x9 mm scaffold, 3 parallelepipeds having dimensions of 9x9x3 mm can be placed into mutual contact.

The scaffold prepared from a single portion or obtained from a plurality of portions may have the form of the bone defect and/or a dimension equal or lower to the bone defect itself.

The preparation of the single portions occurs starting from a commercially available sterile material, which is cut in sterile conditions, for example with a scalpel, to form the desired dimension.

Said scaffolds and said MSC are preferably characterized as reported and described in the appended claims.

The scaffold containing the MSC, and obtained by a plurality of adjacent portions, show an effective vascularization deriving from a synergy between the PRP and the surfaces of the single scaffold portions on which the PRP is arranged.

The subdivision in adjacent portions allows to promote/encourage the vascularization during the generation of the bone tissue.

The PRP is not only arranged on the outer surface, but is capable of distributing itself in the canals formed by the multiple portions forming the scaffold and in the bone tissue forming the wall of the bone defect to be filled.

The invention also relates to a device which comprises said scaffolds, containing said MSC directed to the bone differentiation, according to the features above described. Said scaffolds are treated according to the known freezing techniques which permits a good state of preservation and of their transport.

Therefore, the device includes said scaffold and a safe and temperature-resistant container, which keeps the properties of said scaffolds unchanged.

The invention also relates to a process for the preparation of said scaffolds.

The process includes a step in which the mesenchymal stem cells MSC and a plasma rich in platelets PRP are added to a scaffold.

Advantageously, the mesenchymal stem cells CSM are introduced on the scaffold before the PRP.

Alternatively, the PRP is introduced before the MSC or at the same time.

Preferably, the PRP is reacted with an anti-hemorrhagic and/or calcium gluconate.

The mesenchymal stem cells can be recovered, isolated and prepared following the methodologies known to a person skilled in the art.

In particular, the mesenchymal stem cells are recovered by aspiration from the bone marrow existing in the iliac crest.

The isolation of the cells requires the use of a Ficoll solution in a separation column, so as to separate the cells based on the density gradient.

Subsequently, the effectiveness of the isolation process is determined with the Fluorescence-activated cell-sorting (FACS) method. The selected cells must express the antigens HLA-ABC, CD90 and CD105 on the cell surface and must not express the antigens CD33, CD34 and HLA-DR on the cell surface.

The isolated and selected cells are preferably introduced in a standard growth medium, for example DMEM-LG (BioWhitakker^{®}) to be expanded.

The expanded cells are subjected to an osteogenic induction step.

The induction step (cell differentiation in an osteogenic line) is carried out in an osteogenic medium including, for example, dexametasone and/or ascorbic acid and phosphate glycerol.

The scaffold on which the mesenchymal stem cells MSC and the plasma rich in platelets PRP are introduced is of a collagen type, preferably of type I.

In a preferred embodiment, the scaffold is of the Gingistat^{®} type.

In an embodiment of the present invention, the scaffold appears as a single body.

The PRP is preferably prepared through withdrawal of a small quantity of blood. This is then subjected to a centrifugation, so as to isolate the platelet fraction and the plasma. In an embodiment of the present invention, the PRP is activated with and anti-hemorrhagic and/or with calcium gluconate. The anti-hemorrhagic can contain the active substance batroxobine, for example Botropase^{®}. The anti-hemorrhagic and/or calcium gluconate, in the presence of PRP, forms a gelatinous mass which can be arranged into contact with the scaffold surface.

All the operations above described are carried out in sterile conditions. The scaffold containing MSC is subjected to an analysis for pointing out the presence of microbial contaminations, if any, before its therapeutic use.

An exemplifying embodiment is reported in the example 1.

In an embodiment of the invention, the scaffold includes a plurality of portions.

On each of these single portions, the differentiated cells are introduced.

In this way, the single portions can be implanted in the bone defect so as to position the portions in an adjacent manner and in a mutual contact for defining a structure of a single scaffold.

Alternatively, the single portions can be placed adjacent and in a mutual contact for defining a structure of a single scaffold and, subsequently, being implanted in the bone defect.

In another embodiment of the invention, the scaffold includes a plurality of portions.

On each of these single portions, the differentiated cells and the PRP are introduced.

In this way, the single portions can be implanted in the bone defect adjacent and in a mutual contact for defining a structure of a single scaffold.

Alternatively, the single portions can be placed adjacent and in a mutual contact to define a single scaffold structure and, subsequently, be implanted in the bone defect.

The scaffold according to the invention including PRP on the surface and/or divided in portions, finds a valid application in a therapeutic and/or surgical field, as a medicament.

Preferably, it finds an application in the preparation of an implant for treating a bone or parodontal pathology or for repairing a bone and parodontal defect. The scaffold can be used for the preparation of an implant for repairing or filling a bone defect of a parodontal nature.

The structure and the volume of the scaffold according to the invention, as a single structure or as a sum of single portions, is as a function of the bone defect that one wishes to treat.

The volume of the used scaffold can be lower or equal to the volume of the corresponding bone defect.

Advantageously, the implant obtained with the scaffold subject of the invention can be effectively implanted regardless to the dimension of the bone defect and the scaffold structure. Indeed, it is possible to implant a scaffold with dimensions (intended as volume and/or form) lower than those of the bone defect thanks to the effective and suitable vascularization that the scaffold, together with the PRP, is able to promote.

A good vascularization has been ascertained in the preparation of a dental implant, wherein the structure of the scaffold, of dimensions between 120 and 130 mm³, contains a concentration of settled cells between 7500 and 8500 MSC/mm³ of the scaffold.

Another aspect of this invention is a method for implanting said scaffold in a bone defect.

The method includes the following steps:
a. arranging, on the sides of the bone defect, a composition, for example in a gelatinous form, consisting of PRP activated with an anti-hemorrhagic substance and/or calcium gluconate,
b. implanting in the bone defect a scaffold in one of the possible embodiments according to the invention.

Preferably, the sides of the bone defect can be pretreated in order to cause the bleeding of the same, for example through the mechanical action of alveolar spoons or rotary burrs.

### Example 1

### Isolation and expansion of mesenchymal stem cells (MSC)

A sample of bone marrow was collected from the iliac crest of four healthy donors.

The collected sample (10 ml) was placed in a suitable container.

The containers were washed many times with a saline phospate buffer (PBS) (LiStarFish^{®}, Milan) in order to maximize the quantity of bone marrow obtained.

The mononuclear cells were isolated with a Ficoll-Hypaque^{®} density gradient (GEHealthCare^{®}, Milan).

The cells were then re-suspended in a Dulbecco modified Eagle medium (DMEM-LG) (LiStarFish^{®}) containing: 10% fetal bovine serum (FBS) (Hyclone^{®}, Logan, UT, USA), 2mM L-glutamine (LiStarFish^{®}), 100 U/ml penicillin, 100 µg/ml streptomycin (LiStarFish^{®}).

Subsequently, the cells thus treated were placed in culture plates (surface 150 cm²) in order to obtain cell cultures having a density of 1.6×10⁵ cells/cm².

The process of the cell culture was repeated 2 times/week.

At the end of the operations above described, a lot of expanded cells is obtained. When the cells arrived to a 80% confluence (a relative high density of cells, reached by propagation, on the surface of and in the plate), the cells were trypsinized and isolated.

### Selection of mesenchymal stem cells with FACS method

The expanded cells were separated with trypsin, washed with PBS and re-suspended in PBS so as to obtain a concentration of 1x10⁶ CSM/100 µl.

The cells were incubated for 25 minutes in the dark at room temperature with 5 µl of the following antibody solutions:
- anti-CD33 antibody conjugated with phycoerythrin (Chemicon^{®}, Temecula, CA, USA)
- anti-CD34 antibody conjugated with phycoerythrin (Chemicon^{®}, Temecula, CA, USA)
- anti-HLA-DR antibody conjugated with fluorescein-5-isothiocyanate (FITC) (Chemicon^{®}, Temecula, CA, USA)
- anti-HLA-ABC antibody conjugated with (FITC) (Dako^{®}, Glostrup, Denmark)
- anti-CD90 antibody conjugated with phycoerythrin (Chemicon^{®}, Temecula, CA, USA)
- anti-CD105 antibody conjugated with FITC (Chemicon^{®}, Temecula, CA, USA).

The cells were then re-suspended in PBS and analyzed with FACSCalibur^{®} flow cytometer (Becton Dickinson^{®}, San Josè, CA, USA).

The cells expressing on the surface antigens CD90, CD05 and HLA-ABC were positively selected and the cells expressing on the surface antigens CD33, CD34 and HLA-DR were negatively selected.

### Osteogenic differentiation

The cells were seeded in an expansion culture until they reached a sub-confluence but not a 60% confluence. Subsequently, the cells were incubated in an osteogenic medium (OS) including the culture medium above described and also:
- 100 nM Dexametasone (Applichem^{®} GmbH, Darmstadt, Germany)
- 10nM β-glycerol phosphate (Applichem^{®} GmbH, Darmstadt, Germany) and
- 0.05 mM phospate-2-ascorbic acid

After 4 incubation weeks, the osteogenic differentiation was analyzed through immunofluorescence.

The collected samples were incubated with an antibody for the osteopontin (Chemicon^{®}, Temecula, CA, USA) at a concentration of 1:200, overnight, at 4°C. Subsequently, the incubated samples were washed for an hour with a secondary antibody conjugated with FITC and with propidium iodide (2.5 µg/ml).

The analysis was carried out with a laser confocal microscopy (Radiance^{®} 2100; Biorad^{®} Labs., Hercules, CA, USA).

### Microbiological analysis of the MSC cultures

Before introducing the differentiated and expanded MSC cells on the scaffold, it is important to check the presence of microbiological infections.

Analyses for endotoxins, mycoplasma, aerobic and anaerobic bacteria were carried out according to the instructions of the European pharmacopeia.

### Introduction of the MSC cells on the scaffold

The cells above prepared were re-suspended in an expansion medium at a concentration of 5x10⁶ MSC/ml.

A freeze-dried collagen sponge of a type I (Gingistat^{®}, Vebas^{®}, Milan) was cut with a scalpel in sterile conditions in order to form a cube of 5x5x5 mm equal to 125 mm³.

A sample of 1x10⁶ MSC was introduced in the scaffold using a 25 gauge syringe.

The scaffold was incubated for 35 days. Scaffold samples were examined at 14, 21, 28 and 35 days. The sample were washed with PBS, fixed with 4% paraformaldeyde, included in OCT (cryo-embedding matrix), frozen and cut in sections of 10 µm with cryostat. The samples thus prepared can be studied with the common histological techniques. The cut sections were stained with hematoxylin-eosin for evaluating the integrity of the cells and with alizarin red and 1% ammonium hydroxide for evaluating the mineralization of the matrix originating from the MSC. These studies have allowed to show that the MSC distribute themselves within the whole scaffold volume and that the deposition of the bone matrix is uniform throughout the portions of the scaffold itself.

### Example 2

A small blood quantity was collected from a patient having a parodontitis bone defect. This blood collection (60 ml) was subjected to a double centrifugation at 2800 revolutions per 10 minutes, followed by the second one at 3500 revolutions per 15 minutes. With the first centrifugation, the platelets were separated from the remaining corpuscle part of the blood. With the second centrifugation, the platelets were separated from the plasma so as to isolate the platelet fraction and the plasma. The plasma rich in platelets thus obtained was placed on a plate and activated with calcium gluconate (2 cc for 5 cc of PRP). The PRP become a gelatinous mass after a few minute.

The edges of the bone *lacuna* of the patient were previously made bloody by mechanical treatment with a rotary burr.

Said PRP gelatinous mass was placed between the walls of the bone recess and subsequently the bone lacuna was filled with a scaffold according to the invention. After the positioning of the PRP gel, the *gingiva* was sutured according to the normal surgical procedure with an adequate number of suture stitches.

From the results, it can be seen that the reparation of the gingival soft tissues and the vascularization of the bone constantly occurs in a faster and more effective way with the use of PRP.

## Claims

1. Scaffold comprising mesenchymal stem cells (MSC) **characterized in that** it further includes a plasma rich in platelets (PRP).

2. Scaffold according to claim 1, wherein the plasma rich in platelets is arranged on the outer surface of said scaffold.

3. Scaffold according to claim 2, wherein the PRP is reacted with an anti-hemorrhagic and/or calcium gluconate; preferably, the anti-hemorrhagic and/or calcium gluconate are added to PRP in a quantitative/volumetric ratio of 1:2.5.

4. Scaffold according to any one of the claims 1 to 3, wherein said scaffold is made of a collagen material; preferably, collagen of type I.

5. Scaffold according to one or more claims 1 to 4, wherein said MSC are expanded and, subsequently, induced by osteogenic differentiation before being introduced in said scaffold.

6. Scaffold according to one or more claims 1 to 5, wherein said scaffold has pores with a diameter between 100 and 500 microns; preferably, from 250 to 350 microns.

7. Scaffold according to one or more claims 1 to 6, wherein said scaffold has a volume between 100 and 1000 mm³; preferably, from 300 to 900 mm³.

8. Scaffold according to one or more claims 1 to 7, wherein said MSC are present in an initial concentration between 5000 and 10000 MSC/mm³ of scaffold; preferably from 7500 to 8500 MSC/mm³.

9. Scaffold including mesenchymal stem cells (MSC) **characterized in that** it includes a plurality of adjacent portions in mutual contact for defining a single structure.

10. Scaffold according to claim 16, wherein each portion is made of a collagen material; preferably collagen of type I.

11. Scaffold according to claim 9 or 10, wherein said MSC are expanded and, subsequently, induced by osteogenic differentiation before being introduced in said scaffold.

12. Scaffold according to one or more claims 9 to 11, wherein said scaffold has pores with a diameter between 100 and 500 microns; preferably, from 250 to 350 microns.

13. Scaffold according to one or more claims 9 to 12, wherein said scaffold has a volume between 100 and 1000 mm³; preferably, from 300 to 900 mm³.

14. Scaffold according to one or more claims 9 to 13, wherein said MSC are present in an initial concentration between 5000 and 10000 MSC/mm³ of scaffold; preferably from 7500 to 8500 MSC/mm³.

15. Scaffold including a plurality of portions according to one or more claims 9 to 14, **characterized in that** it further includes a plasma rich in platelets (PRP).

16. Scaffold according to claim 15, wherein the plasma rich in platelets is arranged on the outer surface of said scaffold.

17. Scaffold according to claim 15 or 16, wherein the PRP is reacted with an anti-hemorrhagic and/or calcium gluconate; preferably, the anti-hemorrhagic and/or calcium gluconate are added to PRP in a quantitative/volumetric ratio of 1:2.5.

18. Process for the preparation of a scaffold according to one or more claims 1-8, which includes at least a step in which the mesenchymal stem cells (MSC) and a plasma rich in platelets are added to a scaffold.

19. Process for the preparation of a scaffold including a plurality of portions according to one or more claims 9 to 18, which includes at least a step in which mesenchymal stem cells (MSC) are introduced on said portions and a plasma rich in platelets.

20. Use of a scaffold according to one or more claims 1 to 19 for the preparation of an implant for: the treatment of a bone or parodontal pathology; or for repairing a bone or parodontal defect; or for filling a bone defect of a parodontal nature.
